Europäisches Patentamt

European Patent Office

Office européen des brevets

(11) Publication number: **0 257 392 B1**

(12) **EUROPEAN PATENT SPECIFICATION**

(45) Date of publication of patent specification: **03.04.91**

(51) Int. Cl.5: **G01N 1/00, G01N 1/22, G01M 15/00**

(21) Application number: **87111339.5**

(22) Date of filing: **05.08.87**

(54) Exhaust-gas analyzing device.

(30) Priority: **07.08.86 JP 184143/86**

(43) Date of publication of application: **02.03.88 Bulletin 88/09**

(45) Publication of the grant of the patent: **03.04.91 Bulletin 91/14**

(84) Designated Contracting States: **AT DE FR GB IT LU SE**

(56) References cited:
EP-A- 0 155 793      DE-A- 2 306 903
DE-A- 2 836 787      DE-B- 1 809 670
US-A- 3 593 023      US-A- 4 386 534

PATENT ABSTRACTS OF JAPAN, vol. 3, no. 107 (E-136), 8th September 1979, page 96 E 136 & JP-A-54-85090

(73) Proprietor: **HORIBA, LTD.**
**2 Miyanohigashi-machi Kissyoin**
**Minami-ku Kyoto(JP)**

(72) Inventor: **Kojima, Mitoku**
**51-2, Aza-Sannokubo Miai-cho**
**Okazaki City Aichi Prefecture(JP)**
Inventor: **Kohsaka, Hiroji**
**7-56, 2-chome Nishishibukawa**
**Kusatsu City Shiga Prefecture(JP)**
Inventor: **Matsui, Hisao**
**122-76. Aza-takagi Yasu-cho**
**Yasu-gun Shiga Prefecture(JP)**

(74) Representative: **TER MEER - MÜLLER - STEIN-MEISTER & PARTNER**
**Mauerkircherstrasse 45**
**W-8000 München 80(DE)**

## Description

The present invention relates to an analyzing device of a gas exhausted from cars.

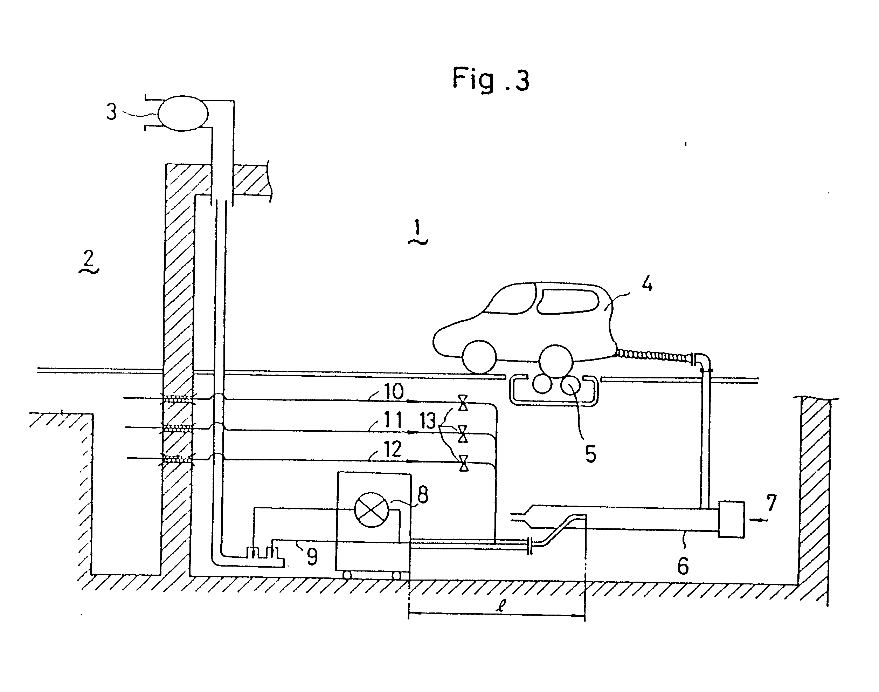

In order to reduce the distance ($\ell$ in the conventional device Fig. 3) from an exhaust-gas analyzer to a sample-inlet of a dilution tunnel for diluting an exhaust gas from cars with air within a sub-atmospheric pressure testing room, the exhaust-gas analyzer has been arranged in the low-pressure testing room. That is to say, a construction as shown in Fig. 3 has been used. In this Fig. 3, the reference numerals shown designate the following elements and features:

    1 = a sub-atmospheric pressure testing room,
    2 = atmospheric pressure,
    3 = a decompression blower,
    4 = a car,
    5 = a chassis dynamometer,
    6 = a dilution tunnel,
    7 = air,
    8 = an analyzer,
    9 = an overflow line,
    10 = a zero gas passage,
    11 = a span gas passage,
    12 = a bag sample passage, and
    13 = a valve.

In the case where HC (hydrocarbon) is measured, if the temperature is reduced, certain kinds of HC change their state of aggregation to other than gas, so that it is necessary to maintain the temperature high and therefore, $\ell$ has been reduced. For example, this is the case where a HC analyzer is used as an exhaust-gas analyzer.

The analytical sensitivity of an exhaust-gas analyzer for use in a car is influenced by pressure, i.e. in the device of Fig.3, the sensitivity depends on the actual pressure within the testing room 1. With conventional analyzers, this problem has not been realized and no measures have been taken for overcoming the above described problem. With measuring methods of this type measurement is carried out only under conditions in which the accuracy is reduced in dependence of the degree of the pressure setting in the low-pressure testing room, or a method has been used, in which the atmospheric pressure is calibrated and then data are read. However, the step of calibrating is difficult, troublesome and time consuming.

It is an object of the present invention to provide an exhaust-gas analyzing device whose sensitivity is influenced by fluctuation of the pressure within a low-pressure testing room less than in conventional devices.

The exhaust-gas analyzing device of the present invention comprises:

    - a dilution tunnel for diluting an exhaust-gas from cars with air of sub-atmospheric pressure,
    - an exhaust-gas analyzer,
    - a pump pumping air from said dilution tunnel into said exhaust-gas analyzer,
    - and an exhaust-pipe, opening to atmospheric pressure, through which the analyzed gas is output from said exhaust-gas analyzer.

With this arrangement the exhaust-gas analyzer always works under atmospheric pressure. In consequence, the sensitivity of the analyzer is less strongly influenced by the pressure fluctuations within the sub-atmospheric pressure testing room.

According to a preferred embodiment, the exhaust-gas analyzer and the pump at its input side are commonly arranged in an analyzing portion.

The inventive concept and embodiments of the invention will be described in the following with reference to the accompanying drawings in which

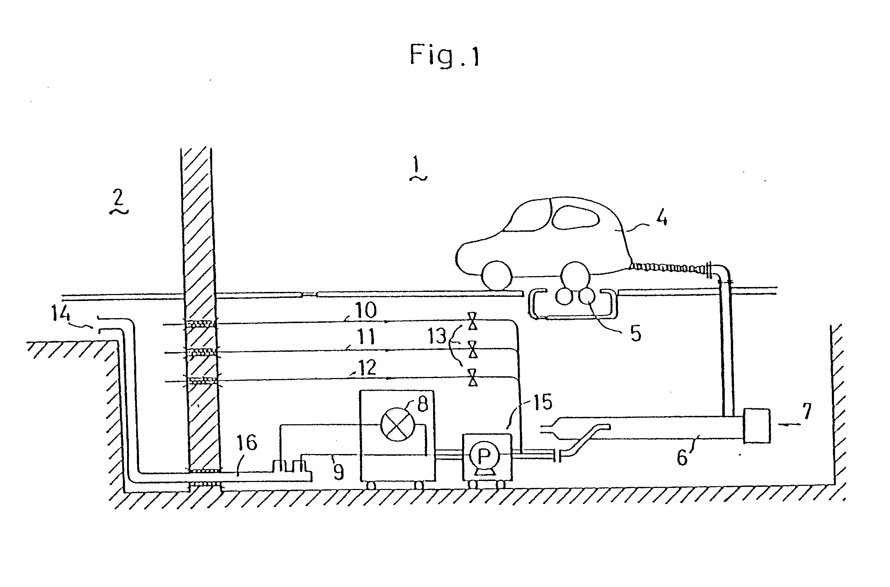

    Fig. 1    is a diagram showing a device according to one preferred embodiment of the present invention,
    Fig. 2    is a diagram showing a device according to another preferred embodiment of the present invention, and
    Fig. 3    is a diagram showing a conventional device.

Referring to Fig. l, reference numerals l, 2, 4 to l3 designate the same members as in Fig. 3 showing the conventional example.

Reference numeral l4 designates an opening of an air side of an exhaust-pipe l6 connected to the analyzer and reference numeral l5 designates a pressure pump provided between the dilution tunnel 6 and the analyzer 8. The exhaust-pipe l6 is guided to the outside of the testing room 1 in an air tight manner.

The operation of the abcve described construction is described in the following.

According to the present invention, since the analyzer 8 works under atmospheric pressure by opening the exhaust-pipe l6 of the analyzer 8 to the atmospheric pressure, the influence of the fluctuation of pressure within the sub-atmospheric pressure testing room upon the accuracy of the analyzer 8 is reduced.

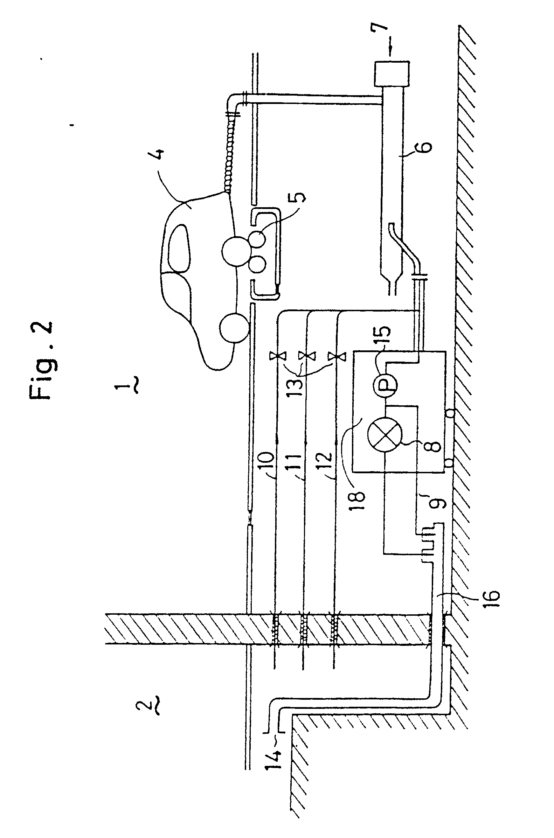

The apparatus of another preferred embodiment of the present invention depicted in Fig. 2 is different from the apparatus of Fig. l merely in that the pressure pump l5 is included in an analyzing portion l8. The operation and effects are the same.

## Claims

1. An exhaust-gas analyzing device in a sub-atmospheric pressure testing room (1) for analyzing gas exhausted from cars (4), said device comprising:

- a dilution-tunnel (6) for diluting an exhaust gas from cars with air (7) of subatmospheric pressure,
- an exhaust-gas analyzer (8) receiving gas from said dilution-tunnel and outputting the analyzed gas through an exhaust-pipe (16),
- and a pump,
  wherein
- said exhaust-pipe (16) opens to air of atmospheric pressure,
- and said pump (15) is arranged at the inlet side of said exhaust-gas analyzer (8) to pump the diluted gas through the analyzer against the atmospheric pressure within the exhaust-pipe (16) and with this within the analyzer.

2. A device as set forth in claim 1, characterized in that said pump (15) and said exhaust-gas analyzer (8) are commonly arranged within an analyzing portion (18).

**Revendications**

1. Dispositif d'analyse de gaz d'échappement dans une chambre d'essai à pression inférieure à la pression atmosphérique pour analyser des gaz d'échappement de véhicule (4), ledit dispositif comprenant :

- un tunnel de dilution (6) pour diluer un gaz d'échappement de véhicule avec de l'air (7) à une pression inférieure à la pression atmosphérique,
- un analyseur de gaz d'échappement (8) recevant du gaz dudit tunnel de dilution et émettant en sortie le gaz analysé au travers d'un conduit d'échappement (16),
- et une pompe,
  dans lequel
- ledit conduit d'échappement (16) débouche à l'air à la pression atmosphérique,
  et ladite pompe (15) est disposée sur le côté entrée dudit analyseur de gaz d'échappement (8) pour pomper le gaz dilué à travers l'analyseur à l'encontre de la pression atmosphérique à l'intérieur du conduit d'échappement (16) et ainsi à l'intérieur de l'analyseur.

2. Dispositif selon la revendication 1, caractérisé en ce que ladite pompe (15) et ledit analyseur de gaz d'échappement (8) sont tous deux disposés dans une partie d'analyse (18).

**Ansprüche**

1. Analysegerät für Kraftfahrzeugabgase in einem Testraum (1), in dem ein geringerer Druck als Atmosphärendruck herrscht, mit folgenden Baugruppen:

- einem Verdünnungsteil (6) zum Verdünnen des Abgases mit luft (7) mit einem Druck unterhalb des Atmosphärendrucks,
- einem Abgasanalysator (8). der Gas aus dem Verdünnungsteil erhäst und das analysierte Gas über ein Abgasrohr (16) ausgibt,
- und einer pumpe,
  wobei
- das Abgasrohr (16) zu luft unter Atmosphärendruck mündet und
- die Pumpe (15) an der Einlaßseite des Abgasanalysators (8) angeordnet ist, um das verdünnte Gas durch den Analysator gegen den Atmosphärendruck im Abgasrohr (16) und damit durch den Analysator zu pumpen.

2. Gerät nach Anspruch 1, dadurch gekennzeichnet, daß die pumpe (15) und der Abgasanalysator (8) gemeinsam in einem Analyseteil (18) angeordnet sind.

Fig.1

# Fig. 2

EP 0 257 392 B1

Fig .3

EP 0 257 392 B1